# EUROPEAN PATENT APPLICATION

(11) **EP 2 124 051 A1**
(43) Date of publication of application: **25.11.2009**
(21) Application number: 08009456.8
(22) Date of filing: 23.05.2008
(51) Int. Cl.: G01N 33/50, G01N 33/68, C12Q 1/37

(54) **Method for rapid generation of phosphorylation profiles, the detection of in vivo phosphorylation sites of kinases and phosphatases and their use as diagnostic markers in cells, tissues and body fluids**

(71) Applicant: ETH Zurich, 8092 Zürich (CH)
(72) Inventor: Aebersold, Ruedi, 8049 Zürich (CH); Bodenmiller, Bernd, 8046 Zürich (CH); Picotti, Paola, 8046 Zürich (CH)

(57) **Abstract**

The invention relates to a new method for the detection and identification of direct and/or indirect phosphorylation/dephosphorylation sites of protein kinases and phosphatases as well as to generate phosphorylation profiles of cells, tissues and body fluids at different physiological states. The such identified phosphorylation/ dephosphorylation sites and phosphorylation profiles and their diagnostic markers can be used to monitor kinase and/or phosphatase activity, to support drug target discovery (e.g. by identifying which kinases/pathways are disregulated in a given diseased state), to classify diseased tissues for optimal treatment and for drug development (e.g. kinase inhibitor screens, off target effect assessment, pharmacokinetics,...). It furthermore comprises the generation of corresponding specific mass spectrometry assays for the detection an quantification of a list or library of identified specific kinase and/or phosphatase targets. Furthermore it pertains to the use of such a selective diagnostic marker and/or of such a mass spectrometry assay, respectively, for the measurement and/or analysis of kinase and/or phosphatase activity, preferentially under differentially influenced conditions by comparison of proteome samples subjected to and not subjected to physiologically/biologically/chemically active compounds. The method in particular includes the following steps: (1a) selective isolation of phosphopeptides from a digest of a reference (sub)proteome to form at least one reference phosphopeptide isolate; (1b) selective isolation of phosphopeptides from a cell state which yields the (sub)proteome of interest (POI). The cell states and therefore the POI can comprise first, a proteome in which a defined kinase or phosphatase gene was knocked-out, knocked down, over expressed or the kinase or phosphatase was inhibited or activated or was temperature sensitive or lacked its activity (kinase dead) or second, a proteome in which a kinase and/or phosphatase or signalling pathway was disregulated (e.g. cancer cells/tissue) or third, a proteome from diseased tissue in which one or several kinases or phosphatases or signalling pathways were disregulated to form at least one phosphopeptide isolate of interest; (2) generation of individual LC-MS(/MS) maps (phosphoproteome patterns) for each phosphopeptide isolate using quantitative (label-free) mass spectrometry; (3) correlation and comparison of the at least one phosphoproteome pattern of the reference phosphopeptide isolate with the at least one phosphoproteome pattern of the POI phosphopeptide isolate and determination of phosphopeptide features showing differential behaviour between the wild-type and the POI phosphopeptide isolate; (4) collecting data and determination of correspondingly regulated phosphopeptide features according to steps (1b)-(3) for at least one further or several or all possible POI(s) (as defined under 1b) (5) the determination of at least one or a list of regulated phosphopeptide feature(s) uniquely regulated by a specific kinase or phosphatase, between cell states (POIs) yielding its selective diagnostic marker(s).

## Description

### TECHNICAL FIELD

The invention relates to a new method for the detection and identification of direct and/or indirect phosphorylation/dephosphorylation sites of protein kinases and phosphatases as well as to generate phosphorylation profiles of cells, tissues and/or body fluids at different physiological states. The such identified phosphorylation/dephosphorylation sites and phosphorylation profiles can be used to monitor kinase and/or phosphatase activity, to support drug target discovery (e.g. by identifying which kinases/pathways are disregulated in a given diseased state), to classify diseased tissues for optimal treatment and for drug development (e.g. kinase inhibitor screens, off target effect assessment, pharmacokinetics,...). It furthermore comprises the generation of corresponding specific mass spectrometry assays for the detection an quantification of a list or library of identified specific kinase and/or phosphatase targets.

### BACKGROUND OF THE INVENTION

Phosphorylation and de-phosphorylation of proteins is catalyzed by enzymes called kinases and phosphatases, respectively, and is the basis of most signalling networks and cascades and critical to the control of most biological processes. As can be anticipated, dysregulation of these enzymes and the consequent effects on the signalling networks and their control have been proven to be responsible for diseases such as cancer or diabetes.In contrast to investigating the phosphorylation state of a single protein, the analysis and understanding of the phosphorylation-pattems on a system-wide scale allows for the identification of central regulators and therefore potential drug targets in the disregulated networks.

Many techniques for determining the phosphorylation state of single, isolated proteins have been developed and successfully applied. Nevertheless, the (time resolved) analysis, particularly if quantitative, of protein phosphorylation based signalling networks or kinase- and phosphatase-substrate networks on a proteome-wide scale poses significant challenges with respect to the selective isolation of the signalling networks, their quantitative analysis and the interpretation of the generated data.

Phosphoproteins challenge the sensitivity of analytical methods because they often have a low natural abundance and less than stoichiometric phosphorylation-site occupancy. In addition, every type of phospho-amino acid has its own chemistry, complicating their isolation and subsequent analysis. In the last few years different methods for the selective isolation of phosphopeptides from complex peptide mixtures produced from whole cell lysates were proposed, all with specific strengths and weaknesses.

The methods commonly used for the selective isolation of phosphopeptides can be grouped into chemical and affinity-based methods.

One chemical method involves the beta-elimination of the phosphate group from phosphoserine and phosphothreonine. The resulting double bond can react with various nucleophilic functional groups, which then allows for the isolation of the phosphopeptides.

A second chemical method allows for the selection of serine-, threonine- and tyrosine-phosphorylated peptides. The phosphopeptides are coupled to a solid-phase support using phosphoramidate chemistry. Non-phosphopeptides are removed by washing, and the phosphopeptides are selectively released and deprotected under acidic conditions.

The affinity-based methods for phosphopeptide isolation include IMAC using Ga(III), Fe(III) or other metal ions, and the use of resins containing titanium dioxide (TiO2) or other metal oxides such as zirconium dioxide. Numerous studies using these resins and showing various degrees of specificity for the isolation of phosphopeptides have recently been published.

WO2004/108948, provides systems, software, methods and kits for detecting and/or quantifying phosphorylated polypeptides and/or acetylated polypeptides in complex mixtures, such as a lysate of a cell or cellular compartment (e.g., such as an organelle).

The methods can be used in high throughput assays to profile phosphoproteomes and to correlate sites and amounts of phosphorylation with particular cell states. Specifically a method for characterizing phosphorylated polypeptides in a sample is proposed comprising : providing a biological sample comprising plurality of polypeptides; digesting the polypeptides with a protease, thereby generating a plurality of test peptides; collecting a fraction of test peptides which are enriched for positively charged peptides; and determining an identifying characteristic of a positively charged peptide in the fraction, wherein collecting the fraction comprises exposing the plurality of test peptides to a strong cation exchanger and wherein peptides are eluted from the strong cation exchanger at pH 3 and those collected which are enriched for phosphorylated peptides.

### SUMMARY OF THE INVENTION

Here we present an invention based on quantitative mass spectrometry which allows to generate comprehensive profiles of multiple or all kinases/phosphatase activities in parallel, to identify diagnostic markers of a given kinase/phosphatase and to monitor its activity using targeted proteomics methods.

The presented invention provides a method to detect, identify an quantify direct and indirect target phosphorylation/dephosphorylation sites of kinases and phosphatases (phosphopeptides, called "diagnostic markers") and/or to generate phosphorylation profiles *in vivo* on a system-wide scale. These targets and/or profiles are then used to quantitatively monitor and assay the activity of the kinases and phosphatases upon different stimuli or conditions, to support drug target discovery by determining e.g. which kinases/phosphatases/pathways are disregulated in a given diseased state, to classify diseased tissues for optimal treatment based on their phosphorylation state, monitor the efficacy of a pharmacological therapy and to support drug development (e.g. kinase inhibitor screens, off target effect assessment, pharmacokinetics,...) Finally, a list of regulated phosphorylation sites, called "diagnostic markers" is given for a set of yeast kinases and phosphatases, disease states, drug treatments etc... The experimental pipeline for the generation of the phosphorylation profiles and the detection of the diagnostic markers is based on highly specific phosphopeptide isolation, (label-free) quantitative phosphoproteomics and computational data analysis. The experimental pipeline for determining the activity of the kinases and phosphatases or to profile the phosphorylation state of cells, tissues or body fluid relies on monitoring quantitatively the (panel) of diagnostic markers using quantitative mass spectrometry assays such as single (multiple) reaction monitoring SRM or label free quantitation. If available, the diagnostic markers can also be detected and quantified using specific antibodies. The overall approach is applicable to all cells from fungi such as yeast (S. cerevisiae) to plantae and animalia like fly (D. melanogaster), mouse (Mus musculus), rat (Rattus rattus) and human (H. Sapiens) from both cell culture as well as tissues and all body fluids. Preferably, all diagnostic markers of phosphorylation profiles are stored as entries in a database.

As concerns the use of the expressions single reaction monitoring (SRM) or multiple reaction monitoring (MRM), the following is to be noted:

Multiple SRM transitions can be measured within the same experiment by rapidly toggling between the different transitions. The term multiple reaction monitoring (MRM) is frequently used to describe such parallel acquisition of SRM transitions, but might be in the future deprecated by the IUPAC nomenclature (current Provisional Recommendations, August 2006).

In the present context the terms are defined as follows:

SRM: Selected reaction monitoring, is a non-scanning mass spectrometry technique, performed on triple quadrupole-like instruments and in which collision-induced dissociation is used as a means to increase selectivity. In SRM experiments two mass analyzers are used as static mass filters, to monitor a particular fragment ion of a selected precursor ion. The specific pair of m/z values associated to the precursor and fragment ions selected is referred to as a "transition" and can be written as parent m/z > fragment m/z (e.g. 673.5 > 534.3). Unlike common MS based proteomics, no mass spectra are recorded in a SRM analysis. Instead, the detector acts as counting device for the ions matching the selected transition thereby returning an intensity value over time.

MRM: Multiple SRM transitions can be measured within the same experiment on the chromatographic time scale by rapidly toggling between the different precursor/fragment pairs (multiple reaction monitoring, MRM). Typically, the triple quadrupole instrument cycles through a series of transitions and records the signal of each transition as a function of the elution time. The method allows for additional selectivity by monitoring the chromatographic coelution of multiple transitions for a given analyte.

Individual or a panel of markers identified are then used to generate molecular probes diagnostic of a cell state and/or can serve as targets for agents that modulate one or more cellular processes. The object of the present invention is therefore in particular a method for the detection and identification of direct and/or indirect targets of kinases and phosphatases or to generate phosphorylation profiles of cells, tissues or body fluids an to use this information for the development of specific assays to probe the activity state of kinases or panels of kinases (phosphatases) in different types of (biological) samples. The method preferentially includes the following steps:
(1a) selective isolation of phosphopeptides from a digest (preferentially the digest being prepared by using a protease such as serine proteases, threonine proteases, cysteine proteases, aspartic acid proteases, metalloproteases, glutamic acid proteases or the like or mixtures of these systems, in particular trypsin, or means of chemical cleavage or peptide bonds or physical peptide bond breakage) of a reference (sub)proteome to form at least one reference (e.g. wild-type) phosphopeptide isolate;
(1b) selective isolation of phosphopeptides from a cell/tissue/body fluid state which yields the (sub)proteome of interest (POI). The cell/tissue/body fluid states and therefore the POI can comprise first, a proteome in which a defined kinase or phosphatase gene was knocked-out, knocked down, over expressed or the kinase or phosphatase was inhibited or activated or was temperature sensitive or lacked its activity (kinase dead) or second, a proteome in which a kinase and/or phosphatase was modulated (activated or inhibited) by chemical inhibition or third, a proteome from diseased tissue in which one or several kinases or phosphatases or signalling pathways were disregulated to form at least one phosphopeptide of the POI.
(2) generation of individual LC-MS(IMS) maps (phosphoproteome patterns) for each phosphopeptide isolate using quantitative (label-free) mass spectrometry;
(3) correlation and comparison of the at least one phosphoproteome pattern of the reference (e.g. wild-type) phosphopeptide isolate with the at least one phosphoproteome pattern of the POI phosphopeptide isolate and determination of phosphopeptide features showing differential behaviour between the wild-type and the POI phosphopeptide isolate;
(4) collecting data and determination of correspondingly regulated phosphopeptide features according to steps (1b)-(3) for at least one further or several or all possible POI(s) (as defined under 1b)
(5) the determination of at least one or a list of regulated phosphopeptide feature(s) uniquely regulated by a specific kinase or phosphatase, between cell states (POIs) yielding its selective diagnostic marker(s).

In point (1) for the specific isolation of phosphopeptides from protein digest either chemical methods can be used. These include beta-elimination of the phosphate group from phosphoserine and phosphothreonine. The resulting double bond can react with various nucleophilic functional groups, which then allows for the isolation of the phosphopeptides. A second chemical method allows for the selection of serine-, threonine- and tyrosine-phosphorylated peptides. The phosphopeptides are coupled to a solid-phase support using phosphoramidate chemistry. Non-phosphopeptides are removed by washing, and the phosphopeptides are selectively released and deprotected under acidic conditions.

Furthermore affinity-based methods for phosphopeptide isolation include immobilized metal affinity chromatography (IMAC) using Ga(III), Fe(III) or other metal ions, and the use of resins containing titanium dioxide (TiO₂) or other metal oxides such as zirconium dioxide or binders such as antibodies or ankyrin repeats can be applied.

As the identification of the phosphopeptides is normally a necessary prerequisite for the SRM(MRM) analysis, the regulated phosphopeptide features should be identified either using tandem mass spectrometry or by inference including former knowledge (based on mass to charge ratio (m/z), accurate mass and retention time in a chromatographic system). This can happen either after step (3) or within step (5) and can be done using directed LC-MS/MS methods.

It is therefore also possible to without any specific knowledge of the actual system behind the feature to carry through the method steps up to step (5) and then only within this step to finally associate features with phosphopeptides influenced by a specific cell state yielding the POI (see point 1b and 4) once the features have proven to be uniquely characterising for a specific system.

According to a first preferred embodiment of the method according to the invention, after step (5) specific mass spectrometry assays based on LC-SRM(MRM) and/or LC/MS and/or LC-MS/MS are developed for the selective detection and quantification of the diagnostic markers. Preferably in this step in multiple reaction monitoring analyses two mass filters are used to isolate a (phospho)peptide ion and a corresponding phosphopeptide fragment ion. It is further preferred if the signal of the fragment ion is then monitored over the chromatographic elution time. Generally, quantitation can be achieved using areas or height of the resulting multiple reaction monitoring peak, optionally after normalization with internal standards, using label free approaches or based on isotope labelling. Peptide and fragment ion masses and the expected peptide elution time can be determined as marker coordinates defining a specific multiple reaction monitoring assay for a diagnostic marker.

In this context it is preferred if the markers in the multiple reaction monitoring are defined by their relative retention time in a chromatographic system, their peptide mass over charge ratio and the mass over charge ratios of the peptide fragment ions upon peptide fragmentation using preferentially CID or ETD.

In addition the diagnostic markers can also be detected and quantified by means of specific binders such as antibodies or ankyrin repeats against the diagnostic markers using e.g. western plots or ELISA experiments.

For the generation of a complete assay it is, according to a preferred embodiment of the method according to the invention, advised to analyse in step (4) at least one, preferably at least 2, more preferably at least 5, more preferable at least 10, most preferably at least 20 or even a larger number like 50 different or finally all possible POI (e.g. all possible kinase and phosphatase genes knocked out) until a single or a panel of diagnostic markers is found to be highly sensitive in distinguishing between or characterizing for each POI. Correspondingly a list of phosphopeptide features or phosphopeptides (if the attribution of features to corresponding phosphopeptides is already done) corresponding to the targets of each kinase or phosphatase (Preferably this is followed or supplemented by generation of a system-wide kinase/phosphatase-target network)or being unique to a POIs generated.

A further preferred embodiment of the invention is **characterised in that** in the step of re-analysis of the regulated phosphopeptide features and identification of the corresponding phosphopeptides existing database information is used for the identification and/or directed LC-MS/MS for the re-analysis. For the details how the systems are used in this context general reference is made to the more detailed specifications given below. These methods are however not limited to the further specifics of the examples discussed below.

The method as given above involves the selective isolation of phosphopeptides from a digest This selective isolation of phosphopeptides from a digest can be obtained from an extract from cells derived from cell culture, from tissues or from body fluids of any organism of any cell state. It is preferred that three or more biological replicates are analysed for each type of isolate to allow statistical analysis.

Typically within this method the POI of interest is analysed. In the POI one or several kinase(s) or phosphatase(s) either has/have a reduced or lost activity or has/have an increased activity. Therefore the POIs include cells in which 1) a defined kinase or phosphatase gene was knocked-out, e.g. by gene deletion, knocked down (using miRNA or siRNA), inactivated by mutation (kinase dead or temperature sensitive mutant) or over expressed (e.g. by mutation of the gene regulatory regions) 2) the kinase or phosphatase was inhibited or activated by biological or chemical means such as kinase inhibitors or activators 3) diseased tissue(s) or body fluid in which one kinase or phosphatase or several or parts or complete signalling pathway(s) was/were disregulated (e.g. cancer tissue).

According to a further embodiment of the invention, the phosphopeptide isolation in step (1) is carried out using either affinity chromatography such as immobilized metal affinity chromatography and/or titanium dioxide, binders such as antibodies or ankyrin repeats and/or exploiting chemical methods such as beta-elimination or phosphoramidate chemistry.

Rapid application of the method is possible if the comparison and correlation in step (3) is automatically carried out using at least one data-processing machine, preferably by using algorithms such as SuperHirn and Corra (see specific discussion below).

Preferably, in step (5) the data are automatically integrated using at least one data-processing machine and at least one, preferably several different statistical tools are applied to determine phosphopeptides or phosphopeptide features uniquely regulated by a kinase or phosphatase or in POI. In this step therefore features associated with a specific POI are analysed, e.g. with respect to their influence and correlation to different kinase or phosphatase activities or different cancer stages. Those features are finally selected which are essentially not influenced by any other kinase or phosphatase activity and which are therefore uniquely and solely characterising a POI (e.g. the activity of a specific kinase and/or phosphatase, and/or of signalling pathways and/or signalling (sub)networks and/or different diseased tissues) in a sensitive way, i.e. with a strong amplitude. One can therefore say that in this step a set of orthogonal features is determined for a POI, and if not done yet before, the corresponding phosphopeptides are associated to these features.

The invention furthermore pertains to the selective diagnostic marker or list of selective diagnostic markers determined using a method as outlined above.

In addition to that, the invention pertains to a mass spectrometry assay determined using a method as outlined above.

The present invention also relates to the use of a method as outlined above for the generation of a list of selective diagnostic markers for a POI, e.g. list of identified specific kinase and/or phosphatase targets (phosphopeptides) and/or for the generation of corresponding specific mass spectrometry assays for a list of identified specific kinase and/or phosphatase targets (phosphopeptides) / and/or a set thereof monitoring signalling pathways and/or signalling networks. It also relates to the use of a single or a panel of selective diagnostic marker(s) and/or of a mass spectrometry assay(s) as given above, respectively, for the measurement and/or analysis of kinase and/or phosphatase activity, preferentially under differentially influenced conditions by comparison of proteome samples subjected to and not subjected to physiologically/biologically/chemically active compounds.

Last but not least, the invention pertains to physiologically/biologically/chemically active compound as obtained in a method as defined above.

Further embodiments of the present invention are outlined in the dependent claims.

### SHORT DESCRIPTION OF THE FIGURES

In the accompanying drawings preferred embodiments of the invention are shown in which:
- Figure 1: is a general overview;
- Figure 2: is a graphical illustration of the discovery part of the step of detection of *in vivo* targets of kinases and phosphatases;
- Figure 3: is a graphical illustration of the data analysis part of the step of detection of in vivo targets of kinases and phosphatases;
- Figure 4: is a graphical illustration of the validation and high throughput application of the usage of markers to monitor kinase and phosphatase activity;
- Figure 5: is an overview of the simplified method for phosphopeptide isolation
- Figure 6: a) kinase-substrate network of YCK1 and phosphopeptides which vanished upon deletion of the YCK1 and b) reduced kinase-substrate network for YCK1, all proteins involved in vesicle transport are marked with a star in yellow, YCK1 is known to control this process; and
- Figure 7: in (a) an SRM(MRM) peak corresponding to a diagnostic marker phosphopeptide for the yeast kinase YCK1 is shown; in (b) several SRM(MRM) assays for a set of diagnostic markers for kinase activity monitored during the same analysis (< 1 hour).

### DETAILED DESCRIPTION OF THE PREFERRED EMBODIMENTS

The presented invention provides a method to detect and identify direct and indirect target phosphorylation/dephosphorylation sites of kinases and phosphatases (phosphopeptides, called "diagnostic markers") or to generate phosphorylation profiles *in vivo* on a system-wide scale. These targets or profiles are then used to quantitatively monitor and assay the activity of the kinases and phosphatases upon different stimuli or conditions, to support drug target discovery by determining e.g. which kinases/pathways are disregulated in a given diseased state, to classify diseased tissues for optimal treatment based on their phosphorylation state and drug development (e.g. kinase inhibitor screens, off target effect assessment, pharmacokinetics,...). Finally, a list of regulated phosphorylation sites, called "diagnostic markers" is given for a set of kinases and phosphatases, disease states, drug treatments etc.. The experimental pipeline for the generation of the phosphorylation profiles and the detection of the diagnostic markers is based on highly specific phosphopeptide isolation, (label-free or isotope labelled) quantitative phosphoproteomics and computational data analysis. The experimental pipeline for determining the activity of the kinases and phosphatases or to profile cells, tissues or body fluid relies on monitoring quantitatively the (panel) of diagnostic markers using quantitative mass spectrometry assays such as multiple reaction monitoring SRM(MRM) or label free quantitation. Alternatively, the diagnostic markers can be detected and quantified using specific antibodies (if available).The overall approach is applicable to all (eukaryotic) cells from yeast (S. cerevisiae) to fly (D. melanogaster), mouse (Mus musculus), rat (Rattus rattus) and human (H. Sapiens) from cell culture as well as tissues and all body fluids. Preferably, all diagnostic markers of phosphorylation profiles are stored as entries in a database. Individual or a panel of markers identified are then used to generate molecular probes diagnostic of a cell state and/or can serve as targets for agents that modulate one or more cellular processes.

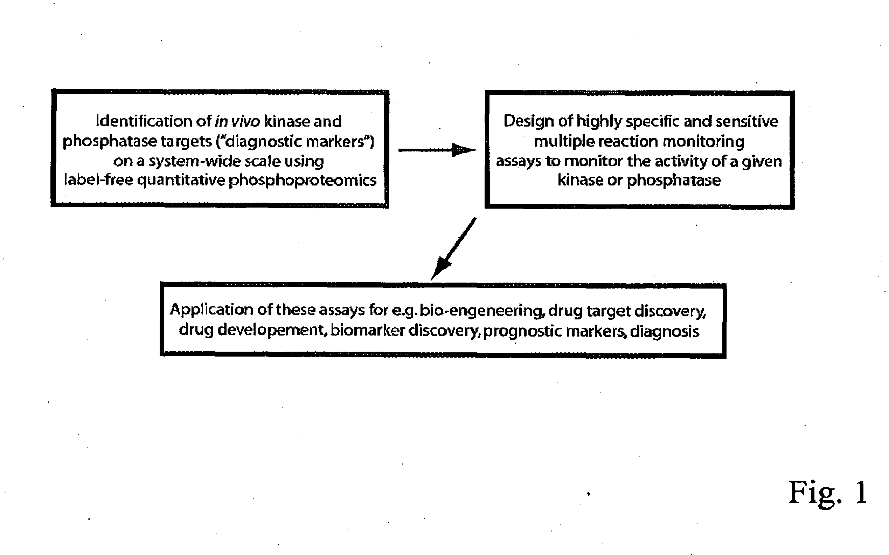

An overview is given in Figure 1.

There are at least four important elements in the present invention, all positioned around the main aspect: the detection and use of phosphopeptides as diagnostic markers to measure kinase and phosphatase activity and/or to profile different cell states.

The first element is that by the application of (label-free) quantitative phosphoproteomics we can identify protein phosphorylation sites which are regulated by a given kinase or phosphatase *in vivo*, on a system-wide scale and in a high-throughput manner, yielding phosphopeptides which can be used as diagnostic markers for kinase/phosphatase activity and/or to profile different cell states.

Second, the design of specific mass spectrometry assays for targeting the diagnostic markers based on multiple reaction monitoring (or label-free quantitative phosphoproteomics using LC-MS/MS) allows to quantitatively monitor in vivo the activity of given kinases/phosphatases (or more general "signalling pathways") under any condition (for any organism). Third, these specific assays can be used in a number of applications, e.g. to detect direct and direct targets of a given kinase or phosphatase, to quantify kinase or phosphatase activity, to detect and quantify disregulated signalling pathways in diseased states as potential drug targets, to use the activity of kinases and phosphatases or signalling pathways to determine the health or disease state (diagnostic or prognostic biomarker discovery), to classify diseased tissues for optimal, personalized treatment, to identify potential drug targets, to optimize drug development by assessing the effect of a drug on a cellular system (including the determination of the point of action, of pharmacokinetics, of structure activity relationships (SAR) and of off target effects), to perform bioengineering. See also further points in Figure 1.

Forth and finally, we provide diagnostic markers for yeast kinases and phosphatases which can be used as assays in order to monitor the kinase/phosphatase activities.

### Technical solution

### a) Detection of in vivo targets of kinases and phosphatases

The experimental pipeline consists of the following steps:
(1a) selective isolation of phosphopeptides from a proteome digest (preferentially the digest being prepared by using a protease such as serine proteases, threonine proteases, cysteine proteases, aspartic acid proteases, metalloproteases, glutamic acid proteases or the like or mixtures of these systems, in particular trypsin or means of chemical cleavage or peptide bonds or physical peptide bond breakage) of a reference (sub)proteome to form at least one reference phosphopeptide isolate;
(1b) selective isolation of phosphopeptides from a cell state which yields the (sub)proteome of interest (POI). The cell states and therefore the POI can comprise first, a proteome in which a defined kinase or phosphatase gene was knocked-out, knocked down, over expressed or the kinase or phosphatase was inhibited or activated or was temperature sensitive or lacked its activity (kinase dead) or second, a proteome in which a kinase and/or phosphatase was modulated (activated or inhibited) by chemical inhibition or third, a proteome from diseased tissue in which one or several kinases or phosphatases or signalling pathways were disregulated to form at least one phosphopeptide of the POI.
(2) generation of individual LC-MS(/MS) maps (phosphoproteome patterns) for each phosphopeptide isolate using quantitative (label-free) mass spectrometry;
(3) correlation and comparison of the at least one phosphoproteome pattern of the reference phosphopeptide isolate with the at least one phosphoproteome pattern of the POI phosphopeptide isolate and determination of phosphopeptide features showing differential behaviour between the reference and the POI phosphopeptide isolate;
(4) collecting data and determination of correspondingly regulated phosphopeptide features according to steps (1b)-(3) for at least one further or several or all possible POI (as defined under 1b).
(5) the determination of at least one or a list of regulated phosphopeptide feature(s) uniquely regulated by a specific kinase or phosphatase, between cell states (POIs) yielding its selective diagnostic marker(s).
(6) Either after step (3) or within step (5) the regulated phosphopeptide features can be preferably re-analyzed and the corresponding phosphopeptides identified using LC-MS/MS or reference peptides.
(7) The data is integrated and different statistical tools are applied to determine phosphopeptides unique to a cell state / POI yielding its diagnostic markers.

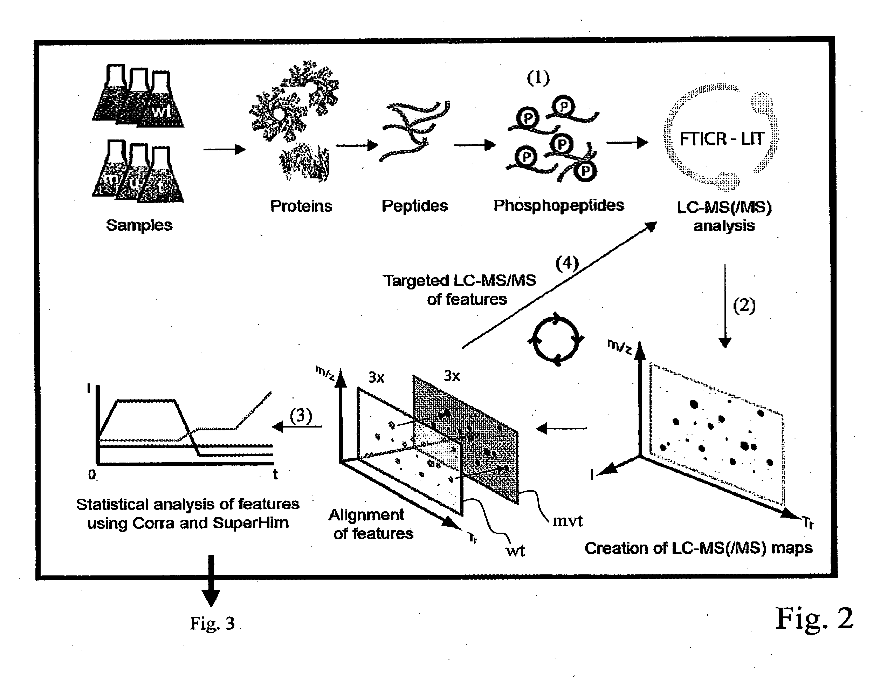

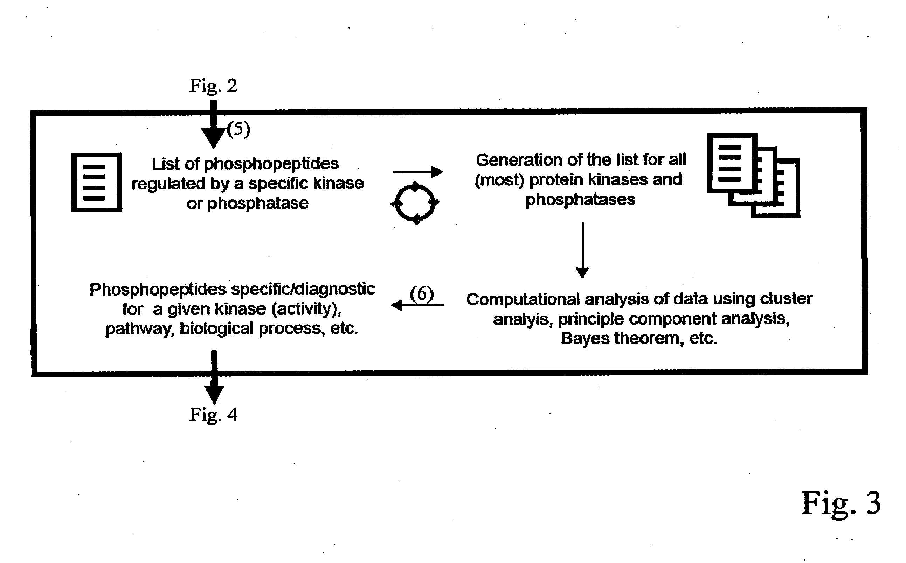

The main elements of this sequence of steps are graphically illustrated in Figure 2 and Figure 3 and the above individual steps (1)-(6) are indicated in the figures.

### b) Use of markers to monitor kinase and phosphatase activity

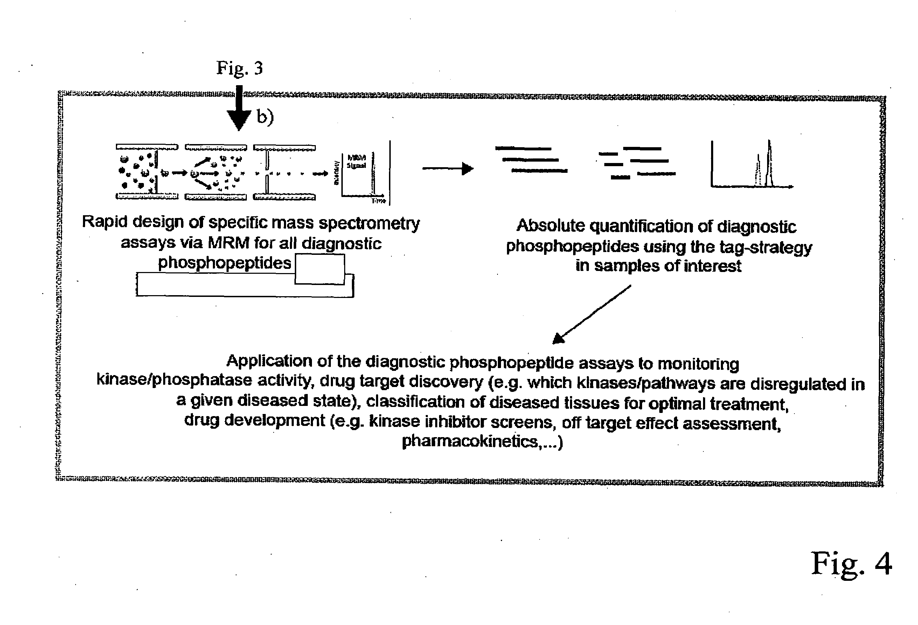

The phosphopeptides (diagnostic markers) identified in a) (or the features if not assigned to phosphopeptides yet) are then monitored by a targeted proteomics approach (see graphical illustration in Figure 4). Specific mass spectrometry assays based on single (multiple) reaction monitoring SRM(MRM) are designed for each diagnostic marker and are used as probes for kinase and phosphatase activity (as an alternative to multiple reaction monitoring also LC/MS or LC-MS/MS based (label-free) quantitation can be used). In SRM(MRM) analyses two mass filters are used to isolate a peptide ion and a corresponding fragment ion.

The signal of the fragment is then monitored over the chromatographic elution time (quantitation is achieved using areas or height of the resulting SRM(MRM) peak, optionally after normalization with internal standards, using label free approaches or based on isotope labelling). Peptide and selected fragment ion masses and the expected peptide elution time are the "marker coordinates" which define a specific SRM(MRM) assay for a diagnostic marker (more specifically the markers are defined by their relative retention time in a chromatographic system, their peptide mass over charge ratio and the mass over charge ratios of the peptide fragment ions upon peptide fragmentation using either CID, ETD or any other fragmentation techniques).

Marker coordinates for each phosphopeptide of interest are extracted from the data acquired under a) (marker peptides and pertaining coordinates can also be derived by bioinformatics prediction by mining existing data repositories or literature resources; SRM(MRM) assays can be optionally validated by triggering full tandem mass spectra, or by coelution of SRM(MRM) peaks associated to different peptide fragments, by SRM(MRM) intensity ratios or by the use of reference peptides) and used to measure by SRM(MRM) the (phospho)peptide of interest, and thus activity of the associated kinase in vivo. With this approach hundreds of diagnostic markers of interest can be quantified in a single analysis (∼ 1 hour).

This allows the high throughput screening of multiple targets in a given POI as well as to screen different POI in a high throughput manner with high sensitivity and accuracy.

Overall this very efficiently, and this is an important aspect of the invention, allows to detect and/or quantify
- Indirect and direct targets of a given kinase or phosphatase,
- Kinase or phosphatase activity,
- disregulated signalling pathways in disease states as potential drug targets,
- to use the activity of kinases and phosphatases or signalling pathways to determine the health or disease state (diagnostic or prognostic biomarker discovery)
- classification of disease tissues for optimal, personalized treatment identify potential drug targets
- monitor the efficacy of a pharmacological therapy
- to optimize drug development by assessing the effect of a drug on a cellular system (including the determination of the point of action, of pharmacokinetics, of structure activity relationships (SAR) and of off target effects). See also further points in Figure 1.
- To optimize bio-engineering processes All steps described under "Technical solution" have been realized in laboratory. Using the approach, a large number of substrates or potential substrates of yeast kinases and phosphatases have been determined and furthermore the effect of drugs on cellular systems has been investigated using the described technology. This shows that the method is can be used in a high throughput manner.

### Advantages of the present invention compared to current technology

One main advantage is that one can generate a list of phosphopeptides which can be used as diagnostic markers to quantitatively monitor kinase and phosphatase activity or to characterize a cell state / POI (see possibilities in other parts of the specification) on nearly system wide scale. This is only possible by the application of label-free quantitative phosphoproteomics via the creation of phosphoproteome patterns which allow monitoring protein phosphorylation events on a cellular wide scale. This makes the phosphorylation pattern approach very suitable for high throughput kinases-target network generation and/or profiling of POIs. Additionally, it results in a sensitivity, specificity, comprehensiveness and speed which are impossible to achieve with a quantitative shotgun proteomics approach.

Second, once the diagnostic markers are determined (coordinates of the markers are stored in databases), the activity of one, several or all kinases or phosphatases (or signalling pathways) can be monitored *in vivo* in a targeted, highly sensitive and high throughput manner using specific multiple reaction monitoring (or label-free quantitative proteomics, both using software which retrieve the coordinates of a given marker) assays.

Third, this experimental pipeline is applicable to POI from all cells, tissues or body fluids.

Fourth, the method and detected diagnostic markers are generally applicable to quantitatively monitor or detect indirect and direct targets of a given kinase or phosphatase,
- Kinase or phosphatase activity,
- disregulated signalling pathways in disease states as potential drug targets,
- to use the activity of kinases and phosphatases or signalling pathways to determine the health or disease state (diagnostic or prognostic biomarker discovery)
- classification of diseased tissues for optimal, personalized treatment
- identify potential drug targets
- monitor the efficacy of a pharmacological therapy
- to optimize drug development by assessing the effect of a drug on a cellular system (including the determination of the point of action, of pharmacokinetics, of structure activity relationships (SAR) and of off target effects). See also further points in Figure 1.
- To optimize bio-engineering processes

### Experimental:

Here we describe a novel method for the system-wide, quantitative profiling of signalling networks using mass spectrometry and data towards establishing the first system-wide kinome-substrate network in *S. cerevisiae, D. melanogaster and H. Sapiens*.

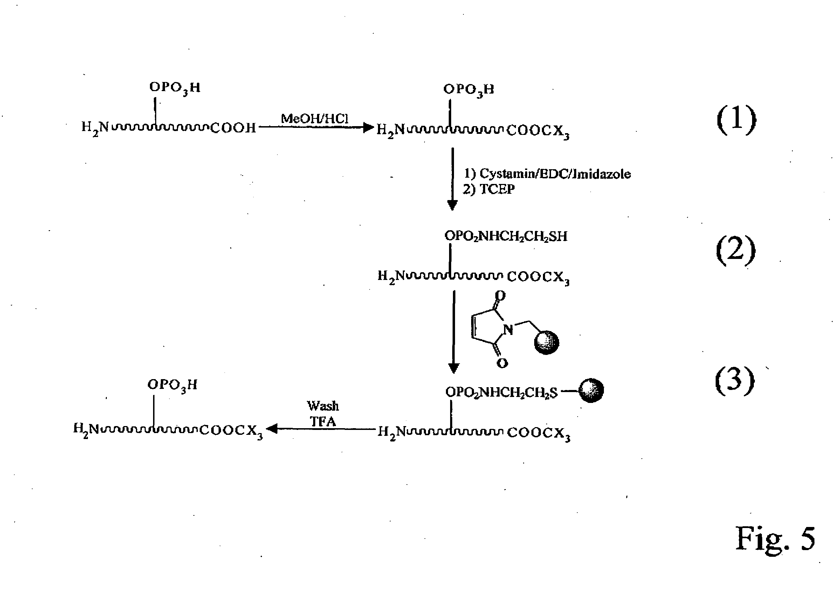

In order to illustrate the depth that can be achieved using phosphoproteomics techniques we applied our strategy to map phosphorylation sites in *D. melanogaster* for which, even though it is an ideal organism for signalling research, any large scale data set on protein phosphorylation was missing. We therefore produced tryptic peptides derived from *D. melanogaster* Kc167 cells which had been exposed to different stimuli. The peptides were fractionated into 42 fractions using isoelectric focusing. Then on each fraction the phosphopeptide isolation methods as follows were applied. With a simplified protocol based on phosphoramidate chemistry we demonstrated a very high specificity for phosphopeptides (up to 90 % purity and yields up to 70 %). In the first step of the reaction pathway (which is schematically illustrated in figure 5) all carboxylate groups are methyl esterified (1). Then the phosphate group is activated (using a carbodiimide) and is coupled to cystamin. Reduction using TCEP yields a free thiol group to couple compound (2) to a maleiimide containing solid phase (3). The solid phase is washed in order to remove non-specifically bound peptides. Finally phosphopeptides are quantitatively released under acidic conditions. As concerns these and other possible methods reference is made to the following publications, the content of which is expressly incorporated into the present specification:
Bodenmiller B, et al,Mol Biosyst. 2007 Apr;3(4):275-86. Epub 2007 Feb 19*.*

Also other phosphopeptide isolation methods (e.g. immobilized metal affinity chromatography (IMAC) and titanium dioxide (TiO₂)) can be used. (See e.g. Bodenmiller B, et al. Nat Methods. 2007 Mar;4(3):231-7. Epub 2007 Feb 11*.)*

Finally all isolates were analyzed by liquid-chromatography tandem mass-spectrometry LC-MS(/MS). As a result of this work we identified in excess of 12,000 phosphorylation sites from over 4,500 phosphoproteins, resulting in the so far most complete phosphoproteome of any system. We could also demonstrate that we achieved an in depth coverage of proteins involved in clinically important signalling pathways such as the insulin/TOR pathway. (see Bodenmiller B, et al . Mol Syst Biol. 2007;3:139. Epub 2007 Oct 16*).*

Overall these results show that the method is capable of detecting system-wide changes in the phosphoproteome upon external stimuli (like drugs). It furthermore allows us to decipher in a time resolved manner which proteins and signalling networks are activated and are therefore important for a given stimulus.

### Generation of an in vivo kinome-substrate network

The experimental details to rapidly generate phosphorylation profiles of cells, tissues and body fluids for the detection of *in vivo* phosphorylation sites of kinases and phosphatases and to monitor their activity are the following (see Figure 1 and 2).

### Example Phosphopeptide isolation

First cells are harvested. In order to preserve their phosphorylation state they are e.g. shock frozen in liquid nitrogen. Then cells are lysed using chemical or physical means and wide range phosphatase inhibitors can be added to the lysis buffer to further preserve the phosphorylation state. After removal of the cell debris, reduction and alkylation of the disulfide bonds, proteins are digested by using by using a protease such as serine proteases, threonine proteases, cysteine proteases, aspartic acid proteases, metalloproteases, glutamic acid proteases or the like or mixtures of these systems, in particular trypsin or means of chemical cleavage or peptide bonds or physical peptide bond breakage) of a reference (sub)proteome to form at least one reference phosphopeptide isolate. After the generation and potential peptide purification using chromatography, phosphopeptides are selectively isolated using methods based on chemical reactions or affinity chromatography. One chemical method involves the beta-elimination of the phosphate group from phosphoserine and phosphothreonine. The resulting double bond can react with various nucleophilic functional groups, which then allows for the isolation of the phosphopeptides. A second chemical method allows for the selection of serine-, threonine- and tyrosine-phosphorylated peptides. The phosphopeptides are coupled to a solid-phase support using phosphoramidate chemistry. Non-phosphopeptides are removed by washing, and the phosphopeptides are selectively released and deprotected under acidic conditions. (*see* Bodenmiller B, et al. Mol Biosyst. 2007 Apr; 3(4):275-86. Epub 2007 Feb 19).

The affinity-based methods for phosphopeptide isolation include IMAC using Ga(III), Fe(III) or other metal ions, and the use of resins containing titanium dioxide (Ti02) or other metal oxides such as zirconium dioxide. Numerous studies using these resins and showing various degrees of specificity for the isolation of phosphopeptides have recently been published. (See Bodenmiller B, et al. Nat Methods. 2007 Mar;4(3)-231-7. Epub 2007 Feb 11.)

Finally antibodies or ankyrin repeats can be used to isolate phosphopeptides with a specific phosphorylation motif or a general class of phosphopeptides such as phosphotyrosine containing peptides. (see Tao WA, et al. Nat Methods. 2005 Aug;2(8):591-8.)

### Example protocols include

*Isolation of phosphopeptides using phosphoramidate chemistry:* 1.5 mg dry peptide was reconstituted in 700 µL of methanolic HCl which was prepared by adding 160 µL of acetyl chloride to 1 mL of anhydrous methanol. The methyl esterification was allowed to proceed at 12 °C for 120 minutes. Solvent was quickly removed in a speedvac, and peptide methyl esters were dissolved 40 µL methanol, 40 µL water and 80 µL acetonitrile (ACN). Then 250 µL of the reaction solution containing 50 mM N-(3-Dimethylaminopropyl)-N'-ethylcarbodiimide (EDC), 100 mM imidazole, pH 6.0, 100 mM 2-(N-Morpholino)ethanesulfonic acid (MES) pH 5.8 and 2 M cystamine were added. Then the pH of the solution was, if necessary, adjusted to pH 5.6. The reaction was allowed to stand at room temperature with vigorous shaking for 8 hrs and then the solution was loaded onto a C18 Sep-Pak cartridge. The derivatized peptides were washed with 0.1 % trifluoroacetic acid (TFA), and then treated with 10 mM TCEP in 100 mM phosphate buffer (pH 6.0) for 8 min in order to produce free thiol groups. After washing with 0.1 % TFA to remove TCEP and phosphate buffer, peptides were eluted with 80 % ACN, 0.1 % TFA. Phosphate buffer was added to adjust final pH to 6.0. Then ACN was partially removed in the speedvac (final concentration of ACN was around 30-40 %) and the derivatized phosphopeptides were incubated with 5 mg maleimide functionalized-glass beads for 1 h at pH 6.2 (Maleimide functionalized glass beads were prepared by 2 hrs reaction between 3 equivalents of 3-maleimidopropionic acid N-hydroxy succinimide ester and 1 equivalent of aminopropyl controlled pore glass (CPG) beads (Proligo Biochemie, Hamburg, Germany)). Then the beads were washed sequentially several times with 3 M NaCl, water, methanol and finally 80 % ACN to remove non-specifically bound peptides. In the last step, the beads were incubated with 20 % TFA, 30 % ACN for 1 h to recover phosphopeptides. The recovered sample was dried in the speedvac and reconstituted in 0.1 % TFA for LC-MS(/MS) analysis.

*Isolation of phosphopeptides using IMAC:* 1.5 mg dry peptide was reconstituted in 700 µL of methanolic HCl which was prepared by adding 160 µL of acetyl chloride to 1 mL of anhydrous methanol. The methyl esterification was allowed to proceed at 12 °C for 120 minutes. Then solvent was quickly removed in a Speedvac. The dry, methylated peptides were reconstituted in 30 % ACN, 250 mM acetic acid at pH 2.7 and mixed with 30 µl equilibrated PHOS-Select™ gel (Sigma-Aldrich) in a blocked mobicol spin column (MoBiTec, Göttingen, Germany). Then the pH of the solution was, if necessary, adjusted to pH 2.65. This peptide solution was incubated for 2 hrs with end-over-end rotation. Then the resin was washed two times with 250 mM acetic acid, 30 % ACN and once with ultra pure water. Finally phosphopeptides were eluted once with 50 mM and once with 100 mM phosphate buffer, pH 8.9 and pH was quickly adjusted to 2.75 after elution. Finally the peptides were cleaned with C 18 ultra micro spin columns (Harvard Apparatus Ltd, Edenbridge, United Kingdom).

*Isolation of phosphopeptides using pTiO₂:* 1.5 mg of peptide was reconstituted in a solution containing 80 % ACN, 2.5 % TFA and was saturated with phthalic acid. Then the peptide solution was added to 4 mg equilibrated TiO₂ (GL Science, Saitama, Japan) in a blocked mobicol spin column (MoBiTec, Göttingen, Germany) and was incubated for 15 min with end-over-end rotation. The column was washed two times with the saturated phthalic acid solution, two times with 80 % ACN, 0.1 % TFA and finally two times with 0.1 % TFA. The peptides were eluted with a 0.3 M NH₄OH solution. If applicable, the recovered peptides were methylated at 12 °C using a similar methanolic HCl to peptide ratio as used for the methylation prior to the isolation of the phosphopeptides using IMAC and PAC. Then peptides were dried and reconstituted in 0.1 % TFA before LC-MS analysis as before

### Generation of phosphorylation patterns

After the isolation of the phosphopeptides individual LC-MS(/MS) maps (phosphoproteome patterns) for each phosphopeptide isolate using quantitative (label-free) mass spectrometry are generated.

### One example protocol is as follows

Samples are analyzed on a hybrid LTQ-Orbitrap mass spectrometer (ThermoFischer Scientific, Bremen, Germany) interfaced with a nanoelectrospray ion source. Chromatographic separation of peptides was achieved on an Eksigent nano LCsystem (Eksigent Technologies, Dublin, CA, USA), equipped with a 11 cm fused silica emitter, 75 m inner diameter (BGB Analytik, Böckten, Switzerland), packed in-house with a Magic C18 AQ 3 m resin (Michrom BioResources, Auburn, CA, USA). Peptides were loaded from a cooled (4°C) Spark Holland auto sampler and separated using ACN/water solvent system containing 0.1% formic acid with a flow rate of 200 nl/min. Peptide mixtures were separated with a gradient from 3 to 35% ACN in 90 min.

Up to three data-dependent MS2 spectra were acquired in the linear ion trap for each FT-MS spectral acquisition range, the latter acquired at 60 000 FWHM nominal resolution settings with an overall cycle time of approximately 1 s. Charge state screening was employed to select for ions with two charges and rejecting ion with one or undetermined charge state. The same sample was injected a second time with the same setting besides the charge state screening, which was then set to three and higher (excluding 1, 2 and undetermined charge state). For injection control, the automatic gain control was set to 5e5 and 1e4 for full FTMS and linear ion trap MS2, respectively. The instrument was calibrated externally according to manufacturers instructions. The samples were acquired using internal lock mass calibration on m/z 429.088735 and 445.120025.

### Phosphorylation pattern comparison

After the generation of the phosphorylation patterns correlation and comparison of the at least one phosphoproteome pattern of the reference phosphopeptide isolate with the at least one phosphoproteome pattern of the POI phosphopeptide isolate and determination of phosphopeptide features showing differential behaviour between the reference and the POI phosphopeptide isolate.

### One example of how the patterns can be compared is given in the following

LC/MS pre-processing, alignment and similarity assessment was performed by the novel program *Superhirn*, which is written in C++ and is freely downloadable (http://tools.proteomecenter.org/SuperHim.php). Initially, *Superhirn* performs a peak detection routine on all input LC/MS runs, where biological signals are separated from background noise. The features are separated from the background noise through a filter which discards all features that do not elute continuously over a minimal time interval. The features are then scored by the product between the feature intensity and the quality of fit to the theoretical isotope distribution pattern. After filtering, the MS1 features are defined by their charge state, mass to charge ratio and apex retention time. Following data pre-processing, retention time fluctuations between two LC/MS runs were removed in an alignment step and LC/MS similarity assessment was performed, which comprises the computation of a LC/MS similarity score and the overlap of MS1 features. The similarity score reflects the reproducibility of intensity and retention time values between common MS 1 features, where the overlap is the percentage of features found in both LC/MS runs. These two steps are repeated for all possible pairs of LC/MS runs and similarity scores as well as feature overlap are represented in a 2D color matrix. Finally, based on the detected and aligned MS1 features, phosphopeptide features that are found to be regulated between the compared phosphorylation patterns can be derived.

### Determination of diagnostic markers

Finally, the data of the patterns of all POIs are integrated and different statistical tools including clustering (e.g. hyerachical), principal component analysis, least square analysis and/or Bayesian interference are applied to determine phosphopeptides unique to a cell state / POI yielding its diagnostic markers.

Therefore this comparative analysis results in the determination of at least one or a list of regulated phosphopeptide feature(s) uniquely regulated by a specific kinase or phosphatase, between cell states (POIs) yielding its selective diagnostic marker(s).

Either after step (3) or within step (5) the regulated phosphopeptide features can be preferably re-analyzed and the corresponding phosphopeptides identified using LC-MS/MS or reference peptides.

The main elements of this sequence of steps are graphically illustrated in Figure 2 and Figure 3.

For illustration of the power of the approach in *S. cerevisiae* quantitative phosphopeptide patterns for all kinase deletion strains were generated and all were analyzed. By using cluster analysis tools based on the SuperHirn (Mueller, L. N.; Rinner, O.; Schmidt, A.; Letarte, S.; Bodenmiller, B.; Brusniak, M. Y., et al. Proteomics 2007, 7, 3470-3480) and Corra (Brusniak, M. Y.; Bodenmiller, B.; Cooke, K.; Campbell, D.; Eddes, J.; Latarte, S., et al. Submitted) software tools, we found that first, the phosphorylation pattern replicates derived from a particular kinase deletion strain were the most related ones. This underlines that the presented experimental pipeline is highly reproducible. Second, for each kinase deletion strain a distinct cluster with dozens to hundreds statistically significant regulated phosphorylation sites were identified, underling that we are able to detect changes in the phosphoproteome upon deletion of a kinase. For example upon the deletion of the yeast kinase yck1 gene we identified over 100 phosphopeptides, about half of them containing the phosphorylation site recognition motifs of this kinase (pS-X-X-S* or (D/E)n>2-X-X-S*), which vanished, indicating that these phosphopeptides were direct targets of the YCK1 kinase.

From these data it can be concluded that the approach allows a system-wide analysis of the kinase-substrate networks in S. cerevisiae and that we are able to identify direct targets of a given kinase. The information contained in such a network - the first one of its kind ever constructed - will be invaluable for the study of signalling systems and regulated processes in general.

### Design of highly specific and sensitive assays for kinase/phosphatase activity based on SRM(MRM)

Towards the design for the highly specific and sensitive assays to monitor kinase and phosphatase activity a mass spectrometric workflow based on multiple reaction monitoring SRM(MRM) is used. SRM(MRM) experiments are performed on triple quadrupole mass spectrometers, by isolating with two mass analyzers a target peptide ion and a fragment ion specific to the peptide of interest. The signals of the fragment ions is then monitored over the elution time. The two-stage mass filtering drastically increases selectivity, and the non-scanning nature of the technique results in higher sensitivity compared to the techniques used in shotgun or label-free proteomics. In our workflow a list of diagnostic markers (phosphopeptides) for a given kinase or phosphatase is selected based on the label-free quantitative approach presented in the previous paragraph. Additionally, phosphorylated peptides of interest to target by SRM(MRM) can be derived by mining existing proteomics data repositories or predicted based on kinase consensus motifs. For each target phosphopeptide, specific fragment ions are derived from the peptide fragmentation pattern and with this information a sample is subjected to an LC-SRM(MRM) analysis that measures the (phospho)peptide of interest, and thus the activity of the associated kinase. In addition, proteins spanning the whole range of protein expression in cells, down to a concentration as low as < 100 copies/cell can be quantified by this approach. Despite its power, SRM(MRM) has so far not been broadly applied in proteomics and especially in phosphoproteomics, mostly due to the difficulty of selecting appropriate diagnostic markers (phosphopeptides) to be used as a target for an SRM(MRM) assay. The previously described platform based on kinase/phosphatase deletion (or inhibition) and label-free quantitative phosphoproteomics overcomes this limitation and allows the fast and high-throughput generation of lists of diagnostic phosphopeptides for SRM(MRM) analysis. With the current instrument control systems and software capabilities, elution times can be used as a constraint for detecting peptides by SRM(MRM), allowing hundreds of peptides of interest to be quantified in a single (∼ 1 hour) LC/MS run. This allows the high throughput screening of multiple targets of the same kinase/phosphatase or to monitor the activity of large numbers of kinases/phosphatases within the same sample.

### Application to measure kinase activity

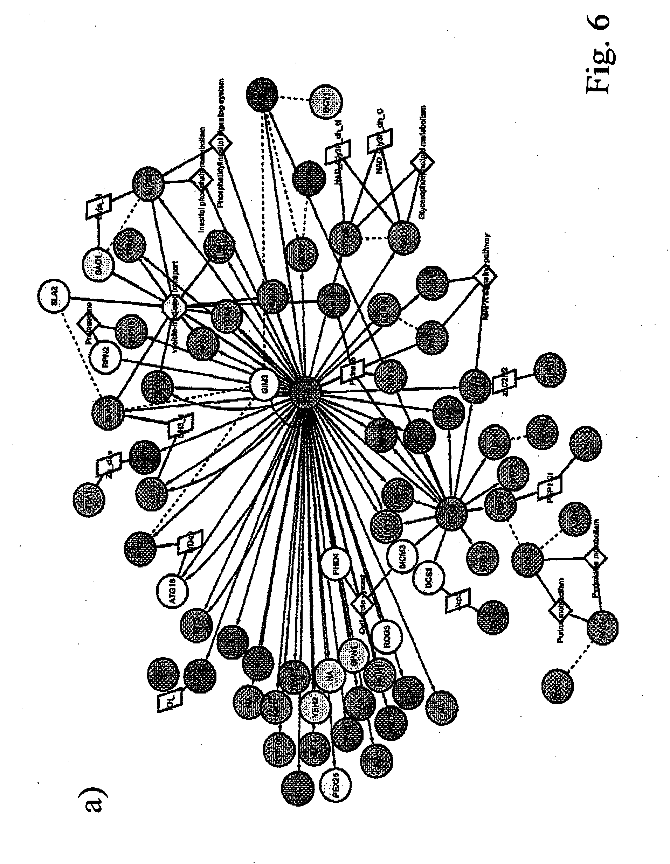

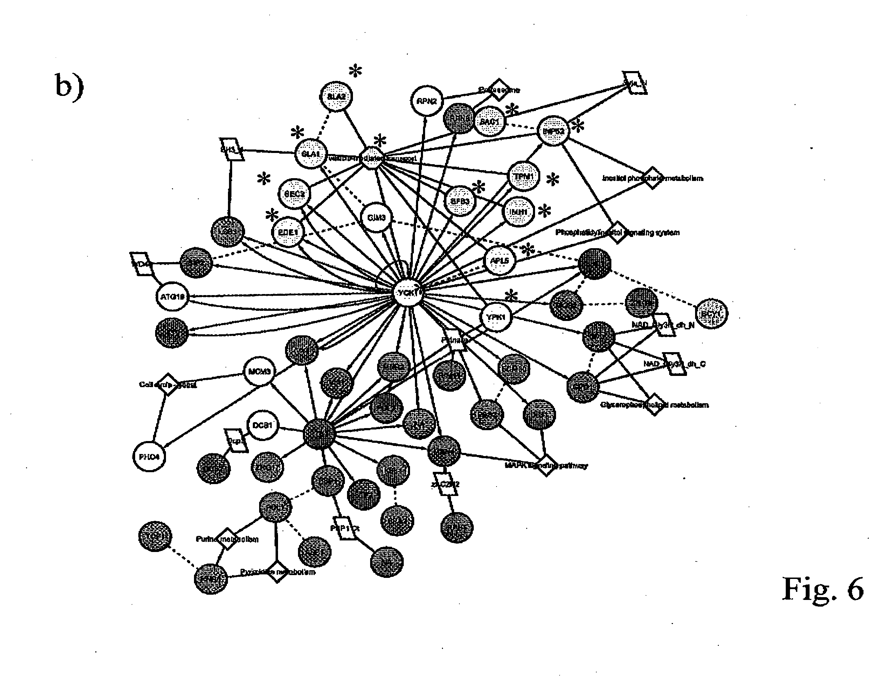

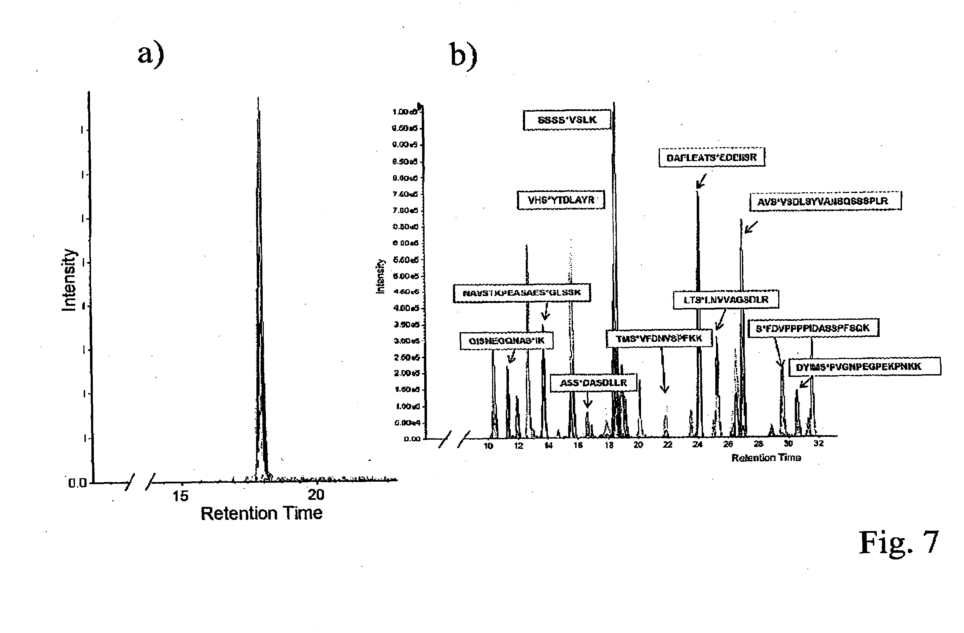

As a proof-of concept demonstration of the presented workflow, we developed SRM(MRM) assays for selected kinase targets which we identified by label-free quantitative phosphoproteomics experiments conducted on wild-type and kinase deletion yeast strains. Marker phosphopeptides were analyzed by SRM(MRM) in phosphopeptide-enriched yeast digests (see Figure 6). Phosphopeptide attributes such as expected retention time, mass-to-charge ratio of the intact peptide and of specific fragment ions, resulting in the highest mass spectrometry signal were chosen as final SRM(MRM) assay for the corresponding kinase. Upon detection of a phosphopeptide SRM(MRM) peak, acquisition of a full tandem mass spectrum was triggered to confirm the identity of the phosphopeptide. Figure 7a shows the successful design of an SRM(MRM) assay for a diagnostic marker of YCK1 kinase activity. Quantitation can be achieved using the SRM(MRM) peak area or height, optionally by normalization using reference peptides. This provides accurate quantitative measure of the phosphopeptide abundance and thus of the activity of the associated kinase. Figure 7b shows the possibility of measuring multiple (up to several hundreds) marker (phospho)peptides in a single (< 1 hour) analysis.

### Coupling protein abundance to phosphorylation site changes

An additional strength of our approach is the possibility, when required, to monitor the change in the degree of phosphorylation of a target phosphoprotein. SRM(MRM) assays can be used and developed to specifically monitor protein abundances in comparison with corresponding phosphopeptide abundances. For a protein of interest a proteotypic peptide (unique to the protein, devoid of phosphorylation sites and good mass spectrometry properties) can be selected by mining proteomics data repositories such as PeptideAtlas. The corresponding SRM(MRM) assay is developed and the peptide quantitatively monitored in an aliquot of the tryptic digest of the proteome of interest, before the sample is subjected to phosphopeptide enrichment. This allows to quantitatively screen for a phosphopeptide and a non-phosphorylated peptide from the same protein, offering the unique possibility to quantitatively discriminate between protein abundance changes and changes in the degree of protein phosphorylation, the latter to be used as a direct measure of the corresponding kinase/phosphatase activity.

Overall we present here a platform which allows identifying kinase and phosphatase targets in a high throughput manner. Furthermore the identified targets are used as "diagnostic" markers to monitor kinase and phosphatase activity under a myriad of conditions, strongly supporting drug target discovery and drug development.

## Claims

1. Method for the detection and identification of direct and/or indirect target phosphorylation/dephosphorylation sites of kinases and phosphatases, respectively, and/or to generate phosphorylation profiles of cells, tissues and body fluids including the following steps:
(1a) selective isolation of phosphopeptides from a digest of a reference proteome to form at least one reference phosophopeptide isolate;
(1b) selective isolation of phosphopeptides from a digest of a proteome of interest to form at least one proteome of interest phosophopeptide isolate,
(2) generation of individual liquid chromatography-mass spectrometry maps and/or liquid chromatography-mass spectrometry/mass spectrometry maps as phosphoproteome patterns for each phosphopeptide isolate using quantitative mass spectrometry;
(3) correlation and comparison of the at least one phosphoproteome pattern of the at least one reference phosophopeptide isolate with at least one phosphoproteome pattern of the at least one proteome of interest phosophopeptide isolate and determination of regulated phosphopeptide features showing differential behaviour between the reference and the proteome of interest phosphopeptide isolate;
(4) collecting data and determination of correspondingly regulated phosphopeptide features according to steps (1b)-(3) for at least one further or several further proteomes of interest,
(5) determination of at least one phosphopeptide feature or a list of regulated phosphopeptide features uniquely regulated characterizing the proteome of interest,
wherein either after step (3) or within step (5) the regulated phosphopeptide features are re-analyzed and the corresponding phosphopeptides are identified yielding the proteome of interest diagnostic markers.

2. Method according to claim 1, wherein the reference proteome is based on healthy and/or wild-type cells, tissues and/or body fluids of an organism and
wherein the proteome of interest, and/or the further proteome(s) of interest, is based on influenced, modified and/or diseased cells, tissues and/or body fluids of an organism, wherein preferably the proteome of interest
is based on a proteome derived from a genome in which a defined kinase or phosphatase or any other gene affecting signalling pathways was knocked-out or mutated, and/or
is based on a proteome in which a kinase or phosphatase gene was knocked down or over expressed, and/or
is based on a proteome in which one or several kinase(s) or phosphatase(s) or another protein were inhibited or activated by a biological, physical or chemical means, preferably by means of preferably specific kinase and/or phosphatase inhibitors or activators such as imatinib mesylate and/or
is based on a proteome the kinase or phosphatase of which was temperature sensitive or lacked its activity, and/or
is based on a proteome from diseased cells or tissues in which any gene or protein affecting signalling pathways was mutated and/or disregulated.

3. Method according to any of the preceding claims, wherein the proteome of interest is based on a system selected from at least one of the following group of systems: eubacteria, archea, bacteria, protista, fungi, preferably *S. cerevisiae* or *S. pombe*, plantae or animalia, preferably *D. melanogaster, M Musculus, R. norvegicus or H Sapiens*.

4. Method according to any of the preceding claims, wherein for quantitation in step (2) label free mass spectrometry and/or isotope labelling dependent quantitation is used.

5. Method according to any of the preceding claims, wherein after step (5) specific mass spectrometry assays based on multiple reaction monitoring and/or LC/MS and/or LC-MS/MS quantitation are developed for the selective diagnostic markers, wherein preferably in multiple reaction monitoring SRM(MRM) analyses two mass filters are used to isolate a peptide ion and a corresponding fragment ion, and wherein the signal of the fragment ion is then monitored over the chromatographic elution time, wherein preferentially quantitation is achieved using areas or height of the resulting multiple reaction monitoring peak, optionally after normalization with internal standards, using label free approaches or based on isotope labelling, and wherein peptide and fragment ion masses and the expected peptide elution time are determined as marker coordinates defining a specific multiple reaction monitoring assay for a diagnostic marker, wherein preferably the markers in the multiple reaction monitoring are defined by their relative retention time in a chromatographic system, their peptide mass over charge ratio and the mass over charge ratios of the peptide fragment ions upon peptide fragmentation using preferentially CID, ETD.

6. Method according to any of the preceding claims, wherein after step (5) binders are raised to detect and quantify the regulated site of phosphorylation, wherein the binders are preferably chemical compounds, most preferably with chemical groups attached which make them detectable with HPLC, NMR, Mass spectrometry and/ or microscopy including fluorescence microscopy such as FACS analysis, and/or wherein the binders are preferably biological compounds, most preferably affinity reagents, preferably antibodies or ankyrin repeats and used in western plot and/or ELISA experiments.

7. Method according to any of the preceding claims, wherein the digest in step (1a) is prepared by using a protease, preferably selected from the group of: serine proteases, threonine proteases, cysteine proteases, aspartic acid proteases, metalloproteases, glutamic acid proteases or mixtures of these systems, in particular trypsin, and/or preferably means of chemical cleavage or peptide bonds or physical peptide bond breakage.

8. Method according to any of the preceding claims, wherein in step (2) and/or in step (4) at least 2, preferably at least 5, more preferably at least 10, most preferably at least 20 different proteomes of interest or even all possible proteomes of interest are analysed to a point that the regulated phosphopeptides are identified as diagnostic markers which distinguish individual proteomes of interest with statistical certainty.

9. Method according to any of the preceding claims, wherein in the step of re-analysis of the regulated phosphopeptide features and identification of the corresponding phosphopeptides existing database information is used for the identification and/or directed LC-MS/MS is used for the re-analysis.

10. Method according to any of the preceding claims, wherein the selective isolation of phosphopeptides from a digest in step (1) is obtained from an extract from cells derived from cell culture, from tissues, preferably from body tissues, and/or from body fluids of an organism, and/or wherein one or two or three or more biological replicates are analysed for each type of isolate.

11. Method according to any of the preceding claims and (1) and (2), wherein the whole cells or proteins on which the phosophopeptide isolates are based are prefractionated before phosphoproteome pattern analysis according to their activation state using chemical physical and/or biological means, preferably fluorescence activated cell sorting such as FACS or specific binders such as affinity reagents like antibodies or ankyrin repeats coupled to beads or another surface.

12. Method according to any preceeding claims, wherein in step (1a) and 1(b) a subproteome is used, preferably
a subproteome which is based or consists of on a part of tissue,
a subproteome which is based on or consists of proteins present in a specific location of a cell such as an organel such as the chloroplast, endoplasmatic reticulum, golgi apparatus, mitochondrion, vacuole and/or the plasma membrane, and/or the cell surface
a subproteome which is based on or consists of proteins and/or their binding partners sharing a function, preferably protein kinases, protein phosphatases, transcription factors, DNA binding proteins, metabolic enzymes, cell surface receptors, proteases, channels, transporters, cell junction proteins and others a subproteome which is based on or consists of proteins and/or their binding partners sharing a biological process like a signal transduction pathway.

13. Method according to any of the preceding claims, wherein the phosphopeptide isolation in step (1) is carried out using either affinity chromatography such as immobilized metal affinity chromatography and/or titanium dioxide, binders such as antibodies or ankyrin repeats and/or exploiting chemical methods such as beta-elimination or phosphoramidate chemistry.

14. Method according to any of the preceding claims, wherein the phosphoproteome patterns generated in step (2) are LC-MS(/MS) maps preferentially measured with an ion trap MS and/or FTICR MS and/or Orbitrap MS and/or TOF(/TOF) MS and/or triple quadrupole MS and/or a combination of these MS types.

15. Method according to any of the preceding claims, wherein the comparison and correlation in step (3) is automatically carried out using at least one data-processing machine, preferably by using algorithms such as SuperHirn and/or Corra, and/or wherein in step (5) the data is automatically integrated using at least one data-processing machine and at least one, preferably several different statistical tools are applied to determine phosphopeptides or phosphopeptide features uniquely regulated in a proteome of interest.

16. Selective diagnostic marker or list of selective diagnostic markers determined using a method according to any of the preceding claims.

17. Mass spectrometry assay determined using a method according to claim 5 possibly in combination with the additional features of any of the preceding claims 6-15 in particular for the identification of a specific kinase and/or phosphatase mutation, knock-out, inhibition, regulation, activation, or over-expression.

18. Use of a method according to any of the preceding claims for the generation of a list of selective diagnostic markers for a list of identified specific kinases and/or phosphatases and/or proteomes of interest and/or for the generation of corresponding specific mass spectrometry assays for a list of identified specific kinases and/or phosphatases.

19. Use of a single or several selective diagnostic marker(s) according to claim 16 for the quantitative measurement of indirect and direct targets of a given kinase or phosphatase or the quantitation of kinase or phosphatase activity or the detection and quantitation of disregulated signalling pathways in diseased states as potential drug targets and/or to determine or to use the quantitatively determined activity of kinases and phosphatases or signalling pathways to determine the health or disease state of a tissue or body fluid to classify diseased tissues for optimal, personalized treatment, monitor the efficacy of a pharmacological therapy, to identify potential drug targets, to optimize drug development by assessing the effect of a drug on a cellular system, including the determination of the point of action, of pharmacokinetics, of structure activity relationships and of off target effects, to perform bioengineering.

20. Physiologically active compound as obtained in a method according to claim 19.
